# EUROPEAN PATENT SPECIFICATION

(11) **EP 2 555 828 B1**
(45) Date of publication and mention of the grant of the patent: **19.07.2017**
(21) Application number: 11711940.4
(22) Date of filing: 05.04.2011
(51) Int. Cl.: A61Q 11/00, A61K 8/365, A61K 8/368

(54) **ORAL CARE COMPOSITIONS**
MUNDPFLEGEMITTEL
COMPOSITIONS DE PROTECTION ORALE

(30) Priority: 09.04.2010 EP 10159444
(43) Date of publication of application: 13.02.2013
(73) Proprietor: Unilever PLC, a company registered in England and Wales under company no. 41424, Greater London EC4Y 0DY (GB); Unilever N.V., 3013 AL Rotterdam (NL)
(72) Inventor: BRADING, Melanie, Gayle, Wirral Merseyside CH63 3JW (GB); GOLDING, Stephen, Wirral Merseyside CH63 3JW (GB); GREEN, Alison, Katharine, Wirral Merseyside CH63 3JW (GB); LITTLEWOOD, David, Thomas, Wirral Merseyside CH63 3JW (GB); SCOTT, Ann, Elizabeth, Wirral Merseyside CH63 3JW (GB)
(74) Representative: Tansley, Sally Elizabeth
(86) International application number: PCT/EP2011/055275
(87) International publication number: WO 2011/124572

(56) References cited:
- EP-A2- 0 514 966
- EP-A2- 1 074 245
- WO-A1-2008/025468
- FR-A1- 2 772 042
- GB-A- 761 186
- GB-A- 824 513
- US-A- 5 098 711
- US-A1- 2005 026 981
- US-A1- 2006 009 469
- US-A1- 2007 110 683

## Description

### Field of the Invention

The present invention is concerned with oral care compositions. More particularly, the present invention is concerned with oral care compositions containing alkyl esters of gallic acid.

### Background of the Invention

Alkyl esters of gallic acid, such as propyl gallate, have antioxidant properties and may be used in oral care compositions to exert beneficial physiological effects, such as prevention or amelioration of gingivitis or periodontitis. JP 04290819 also describes gallic acid and its C1-4 alkyl esters as collagenase inhibitors effective in the treatment of periodontosis, a degenerative, noninflammatory condition of the periodontium.
GB761186A describes antioxidant concentrates to stabilise fats, fatty oils and fatty acids.

However, the incorporation of alkyl esters of gallic acid into oral care compositions (such as toothpastes) presents problems. Degradation and unattractive discoloration have been observed on storage.

The present inventors have found that the above problems can be solved by incorporating a source of citrate ions into the composition.

### Summary of the Invention

The present invention provides an oral care composition comprising an alkyl ester of gallic acid and a source of citrate ions; in which the source of citrate ions is zinc citrate; and in which the amount of zinc citrate ranges from 1 to 3 % by weight (by total weight source of citrate ions based on the total weight of the oral care composition); and in which the amount of alkyl ester of gallic acid ranges from 0.01 to 0.05 % by total weight alkyl ester of gallic acid based on the toal weight of the oral care composition

### Detailed Description of the Invention

Typical alkyl esters of gallic acid for use in the present invention include n-propyl gallate (n-propyl-3,4,5-trihydroxybenzoate) or isopropyl gallate (isopropyl-3,4,5-trihydroxybenzoate) or gallic acid methyl ester (methyl-3,4,5-trihydroxybenzoate) or gallic acid ethyl ester (ethyl-3,4,5-trihydroxybenzoate)- or gallic acid butyl ester (n-butyl-3,4,5-trihydroxybenzoate). The preferred alkyl ester of gallic acid for the purposes of the present invention is n-propyl gallate. Mixtures of any of the above described materials may also be used.

Suitable sources of citrate ions for use in the present invention include citric acid or various cosmetically acceptable soluble or sparingly-soluble citrate salts. Sparingly-soluble citrate salts are preferred, since these may further improve product aesthetics by providing an additional opacifying effect. Soluble citrate salts are defined as at least 1g of citrate salt dissolved per 100 g of solvent at 25° C. Sparingly-soluble citrate salts are defined as from 0.1 to 1g of citrate salt dissolved per 100 g of solvent at 25° C. Since the compositions of the invention are usually aqueous-based, the "solvent" in the above context is usually water. Specific examples of citrate salts useful in the present invention include sodium citrate, ammonium citrate and potassium citrate, with zinc citrate being especially preferred since it is sparingly water-soluble. Zinc ions are also of value for their antiplaque and anti-calculus properties. Mixtures of any of the above described materials may also be used.

Zinc citrate is source of citrate ions for the purposes of the present invention and the amount of zinc citrate ranges from 1 to 3% by weight based on the total weight of the oral care composition.

Further antioxidant compounds may usefully be incorporated into compositions of the present invention, in order to boost performance and/or product stability.

A preferred class of such further antioxidant compounds includes tocopherols and derivatives thereof. The tocopherols are mono, di, and trimethylated derivatives of the parent substance tocol (2-methyl-2-(4,8,12-trimethyltridecyl) chroman-6-ol). The configuration 2R,4'R,8'R is assigned to alpha-tocopherol, which occurs most frequently in nature and is the most important source of vitamin E activity. It is occasionally also called RRR-alpha-tocopherol. The tocopherols and derivatives thereof which are preferred for use in the invention are alpha-tocopherol and its esters, such as alpha-tocopherol succinate, alpha-tocopherol nicotinate and alpha-tocopherol acetate. Alpha-tocopherol acetate is especially preferred.

When present, the amount of tocopherols and/or derivatives thereof suitably ranges from 0.0001 to 10%, preferably from 0.01 to 5%, more preferably from 0.5 to 1.5% by total weight tocopherols and/or derivatives thereof based on the total weight of the oral care composition.

Another preferred class of further antioxidant compounds includes curcumin compounds. Curcumin is a polyphenolic yellow pigment which occurs naturally in the rhizome of the species *Curcuma longa,* which is grown commercially and sold as turmeric, a yellow-orange dye. Curcumin is also known as diferuloylmethane or (1E,6E)-1,7-bis (4-hydroxy-3-methoxyphenyl) hepta-1,6-diene-3,5-dione. Turmeric contains curcumin along with other natural analogues of curcumin which are collectively called "curcuminoids". The major curcuminoids present in turmeric are demethoxycurcumin (also known as *p*-hydroxycinnamoyl(feruloyl)methane), bis-demethoxycurcumin (also known as *p,p'-*dihydroxydicinnamoylmethane) and cyclocurcumin (in which the alpha, beta-unsaturated beta-diketone group of curcumin is replaced by an alpha, beta-unsaturated dihydropyranone group). The term "curcumin compound" for the purposes of the present invention encompasses curcumin, the curcuminoids, other natural or synthetic derivatives or analogues of curcumin, and also preparations of the plant *Curcuma longa* or other curcumin-containing plants, such as *Curcuma xanthorrhiza, Curcuma zedoaria* and *Curcuma aromatica.* Examples of typical curcumin compounds for use in the present invention include curcumin, demethoxycurcumin, bis-demethoxycurcumin, sodium and other alkali metal curcuminates, diacetylcurcumin, triethylcurcumin, dihydrocurcumin, tetrahydrocurcumin, tetrahydrodemethoxycurcumin, tetrahydrobis-demethoxycurcumin, hexahydrocurcumin, octahydrocurcumin, curcumin glucuronide, and curcumin sulphate. Preferred examples are curcumin, demethoxycurcumin, bis-demethoxycurcumin, tetrahydrocurcumin, tetrahydrodemethoxycurcumin and tetrahydrobis-demethoxycurcumin. Mixtures of any of the above described materials may also be used. Especially preferred are mixtures of curcumin, demethoxycurcumin and bis-demethoxycurcumin, and mixtures of tetrahydrocurcumin, tetrahydrodemethoxycurcumin and tetrahydrobis-demethoxycurcumin.

When present, the amount of curcumin compound(s) suitably ranges from 0.0001 to 3%, preferably from 0.01 to 2%, more preferably from 0.5 to 1.5% by total weight curcumin compound(s) based on the total weight of the oral care composition.

Another preferred class of further antioxidant compounds includes ascorbic acid and/or derivatives thereof. The ascorbic acid and/or derivative thereof for use in the present invention may be ascorbic acid and/or any cosmetically acceptable water-soluble or oil-soluble ascorbic acid derivative. The term "ascorbic acid and/or derivative thereof" encompasses ascorbic acid as well as esters of ascorbic acid, and ester salts of ascorbic acid such as ascorbyl phosphates as well as ascorbic acid derivatives such as ascorbyl palmitate, ascorbyl tetraisopalmitate (for example available from DSM), ascorbyl dipalmitate (for example, NIKKOL CP available from Nikko Chemical), ascorbyl linoleate, ascorbyl octanoate, 2-O-D-glucopyranosyl-L-ascorbic acid, which is an ester of ascorbic acid and glucose and usually referred to as L-ascorbic acid 2-glucoside or ascorbyl glucoside, and its metal salts. The term "ascorbyl phosphate" denotes metal salts of mono- and polyphosphoric acid esters of ascorbic acid in which the phosphorylated hydroxy group of the ascorbic acid molecule features one or more phosphoric acid (phosphate) units, and in which metal cations (such as sodium and/or magnesium or calcium ions) are also present. The term "poly" generally denotes 2 to 10, preferably 2 to 4 phosphate units. The ascorbyl phosphates may also be referred to in general as "ascorbyl (poly)phosphates" to embrace both mono- and polyphosphates. Typical ascorbyl phosphates for use in the present invention are L-ascorbic acid phosphate ester salts such as sodium ascorbyl phosphate, potassium ascorbyl phosphate, magnesium ascorbyl phosphate, calcium ascorbyl phosphate and sodium magnesium L-ascorbyl-2-monophosphate. Commercially available ascorbyl phosphates comprise trisodium L-ascorbyl-2-monophosphate which is available as STAY-C®50 form DSM Nutritional Products AG, (4303 Kaiseraugst, Switzerland) and magnesium L-ascorbyl phosphate (available from Showa Denko) and sodium magnesium L-ascorbyl-2-monophosphate. The preferred ascorbyl phosphate for the purposes of the present invention is trisodium L-ascorbyl-2-monophosphate. Mixtures of any of the above described materials may also be used.

When present, the amount of ascorbic acid and/or derivatives thereof suitably ranges from 0.0001 to 10%, preferably from 0.01 to 5%, more preferably from 1 to 3% by total weight ascorbic acid and/or derivatives thereof based on the total weight of the oral care composition.

Mixtures of any of the above described further antioxidant compounds may also be used. Particularly preferred are mixtures including at least two of, and most preferably all of, the following further antioxidant compounds: alpha-tocopherol acetate, ascorbyl phosphate, tetrahydrocurcumin, tetrahydrodemethoxycurcumin and tetrahydrobis-demethoxycurcumin (in suitable individual amounts such as those specified above according to the class of antioxidant).

The total amount of further antioxidant compound(s) suitably ranges from 0.0001 to 15%, preferably from 0.01 to 5%, more preferably from 2 to 4% by total weight further antioxidant compound(s) based on the total weight of the oral care composition.

The oral care compositions of the invention may be in any form common in the art, for example a dentifrice, a mouthwash, gum or lozenge.

A preferred type of product form in the context of the present invention is a dentifrice. The term "dentifrice" denotes paste, cream, mousse, aerosol, powder, gel, and/or liquid formulations which are used to clean the surfaces of the oral cavity. The dentifrice is an oral composition that is not intentionally swallowed for purposes of systemic administration of therapeutic agents, but is retained in the oral cavity for a sufficient time to contact substantially all of the dental surfaces and/or mucosal tissues for purposes of oral activity. Preferably the dentifrice is in the form of a paste or a gel (or a combination thereof).

A dentifrice composition according to the invention will generally contain further ingredients to enhance performance and/or consumer acceptability such as abrasive cleaning agent, water, humectant, binder or thickening agent, and surfactant.

For example, a dentifrice will usually comprise an abrasive cleaning agent in an amount of from 3 to 75% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Furthermore, the dentifrice will usually contain a liquid phase in an amount of from 40 to 99% by weight based on the total weight of the dentifrice. Such a liquid phase typically comprises water and a humectant in various relative amounts, with the amount of water generally ranging from 10 to 45% by weight (based on the total weight of the dentifrice) and the amount of humectant generally ranging from 30 to 70% by weight (based on the total weight of the dentifrice). Typical humectants include glycerol, sorbitol, polyethylene glycol, polypropylene glycol, propylene glycol, xylitol (and other edible polyhydric alcohols), hydrogenated partially hydrolyzed polysaccharides and mixtures thereof.

Furthermore, the dentifrice will usually contain a binder or thickening agent in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, the dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Compositions of the present invention may also contain further optional ingredients customary in the art such as fluoride ion sources, anticalculus agents, buffers, flavouring agents, sweetening agents, colouring agents; opacifying agents, preservatives, antisensitivity agents and antimicrobial agents.

The invention is further illustrated with reference to the following, non-limiting Examples.

### EXAMPLES

Compositions were prepared having ingredients as shown in Table 1 below.

All ingredients are expressed by weight percent of the total formulation, and as level of active ingredient.

Example A is a comparative example (not according to the invention). Example 1 is a formulation according to the invention.

**Table 1**

| Ingredient | Example 1 | Example A |
|---|---|---|
| Sorbitol | 45 | 45 |
| Sodium fluoride | 0.32 | 0.32 |
| Sodium saccharin | 0.25 | 0.25 |
| PEG-32 | 2.0 | 2.0 |
| Zinc citrate | 2.0 | - |
| Titanium dioxide | 1.0 | 1.0 |
| Hydrated silica (Tixosil®43, ex Rhodia) | 8.5 | 8.5 |
| Hydrated silica (Sorbosil®AC77, ex PQ Corp.) | 10 | 10 |
| Sodium lauryl sulphate | 1.8 | 1.8 |
| Flavour | 1.2 | 1.2 |
| Carboxymethylcellulose, sodium salt (Cekol®2000, ex CP Kelco) | 1.1 | 1.1 |
| THC(TETRAHYDROCURCUMINOIDS CG, ex Sabinsa Corp.)* | 1.0 | 1.0 |
| Propyl gallate | 0.03 | 0.03 |
| Tocopheryl acetate | 1.0 | 1.0 |
| Sodium ascorbyl phosphate (Stay-C® 50, ex DSM) | 1.0 | 2.0 |
| Water | to 100 | To 100 |

| | | |
|---|---|---|
| * A mixture of tetrahydrocurcumin, tetrahydrodemethoxycurcumin and tetrahydrobis-demethoxycurcumin extracted from the roots of the *Curcuma longa* (turmeric) plant. | | |

After 4 months storage at elevated temperature (40°C), the formulations were tested for propyl gallate levels. The percentage of propyl gallate recovered from the formulation of Example 1 was 46.7%. By contrast, the percentage of THC recovered from the formulation of Example A was 1.3%, indicating greater propyl gallate degradation in the formulation of Example A.

## Claims

1. An oral care composition comprising an alkyl ester of gallic acid and a source of citrate ions; in which the source of citrate ions is zinc citrate; and in which the amount of zinc citrate ranges from 1 to 3% by weight (based on the total weight of the oral care composition), and in which the amount of alkyl ester of gallic acid ranges from 0.01 to 0.05% by total weight alkyl ester of gallic acid based on the total weight of the oral care composition.

2. An oral care composition according to claim 1, in which the alkyl ester of gallic acid is n-prupyl gallate.

3. An oral care composition according to any preceding claim, which is in the form of a dentifrice and which comprises abrasive cleaning agent, water, humectant, binder or thickening agent. and surfactant.

## Patentansprüche

1. Mundpflegezusammensetzung, umfassend einen Alkylester der Gallussäure und eine Citrationen-Quelle, worin die Citrationen-Quelle Zinkcitrat darstellt und in welcher die Menge des Zinkcitrats zwischen 1 und 3 Gewichts-% (bezogen auf das Gesamtgewicht der Mundpflegezusammensetzung) liegt und worin die Menge des Alkylesters der Gallussäure zwischen 0,01 und 0,05%, in Gesamtgewicht des Alkylesters der Gallussäure, bezogen auf das Gesamtgewicht der Mundpflegezusammensetzung, liegt.

2. Mundpflegezusammensetzung nach Anspruch 1, worin der Alkylester der Gallussäure n-Propylgallat darstellt.

3. Mundpflegezusammensetzung nach irgendeinem vorhergehenden Anspruch, die in Form eines Zahlputzmittels vorliegt und welche abrasives Reinigungsmittel, Wasser, Feuchthaltemittel, Binder oder Verdickungsmittel und Tensid umfasst.

## Revendications

1. Composition de soin oral comprenant un gallate d'alkyle et une source d'ions citrate ; dans laquelle la source d'ions citrate est le citrate de zinc ; et dans laquelle la quantité de citrate de zinc est comprise entre 1 et 3 % en masse (rapportée à la masse totale de la composition de soin oral), et dans laquelle la quantité de gallate d'alkyle est de 0,01 à 0,05 % en masse totale de gallate d'alkyle rapportée à la masse totale de la composition de soin oral.

2. Composition de soin oral selon la revendication 1, dans laquelle le gallate d'alkyle est le gallate de n-propyle.

3. Composition de soin oral selon l'une quelconque des revendications précédentes, laquelle est dans la forme d'un dentifrice et laquelle comprend un agent nettoyant abrasif, de l'eau, un humectant, un liant ou agent épaississant, et un tensioactif.
